# EUROPÄISCHE PATENTANMELDUNG

(11) **EP 1 538 213 A2**
(43) Veröffentlichungstag der Anmeldung: **08.06.2005**
(21) Anmeldenummer: 04020495.0
(22) Anmeldetag: 28.08.2004
(51) Int. Cl.: C12P 13/08

(54) **Verfahren zur fermentativen Herstellung von L-Aminosäuren unter Verwendung coryneformer Bakterien**

(30) Priorität: 26.09.2003 DE 10344739
(71) Anmelder: Degussa AG, 40474 Düsseldorf (DE); FORSCHUNGSZENTRUM JÜLICH GMBH, 52425 Jülich (DE)
(72) Erfinder: Sindelar, Georg, Dr., 41564 Kaarst (DE); Wendisch, Volker F., Dr., 52428 Jülich (DE); Sahm, Hermann, Prof. Dr., 52428 Jülich (DE)

(57) **Zusammenfassung**

Die Erfindung betrifft ein Verfahren zur Herstellung von L-Aminosäuren, bei dem man folgende Schritte durchführt:
a) Fermentation der die gewünschte L-Aminosäure produzierenden coryneforme Bakterien, in denen mindestens eines oder mehrere der mit dem Stickstoff-Metabolismus verknüpften Gene, ausgewählt aus der Gruppe amt, ocd, soxA und/oder sumT verstärkt, insbesondere überexprimiert oder auf hohem Niveau exprimiert ist (sind).
b) Anreicherung der gewünschten L-Aminosäure im Medium oder in den Zellen der Bakterien, und
c) Isolierung der L-Aminosäure,
und gegebenenfalls Bakterien, in denen man zusätzlich weitere Gene des Biosyntheseweges der gewünschten L-Aminosäure verstärkt, in denen die Stoffwechselwege zumindest teilweise ausgeschaltet sind, die die Bildung der gewünschten L-Aminosäure verringern, einsetzt.

## Beschreibung

Gegenstand der Erfindung ist ein Verfahren zur fermentativen Herstellung von L-Aminosäuren, insbesondere L-Lysin, unter Verwendung coryneformer Bakterien, in denen eines oder mehrere der Gene, ausgewählt aus der Gruppe amt, ocd, soxA und/oder sumT verstärkt ist (sind). Die genannten Gene kodieren für Proteine aus dem Stickstoff-Metabolismus.

### Stand der Technik

Chemische Verbindungen, mit denen insbesondere L-Aminosäuren, Vitamine, Nukleoside und Nukleotide und D-Aminosäuren gemeint sind, finden in der Humanmedizin, in der pharmazeutischen Industrie, in der Kosmetik, in der Lebensmittelindustrie und in der Tierernährung Anwendung.

Zahlreiche dieser Verbindungen werden durch Fermentation von Stämmen coryneformer Bakterien, insbesondere Corynebacterium glutamicum, hergestellt. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder auxotroph für regulatorisch bedeutsame Metabolite sind und L-Aminosäuren produzieren.

Seit einigen Jahren werden ebenfalls Methoden der rekombinanten DNA-Technik zur Stammverbesserung L-Aminosäure produzierender Stämme von Corynebacterium glutamicum eingesetzt, indem man einzelne Aminosäure-Biosynthesegene amplifiziert und die Auswirkung auf die L-Aminosäure-Produktion untersucht.

### Aufgabe der Erfindung

Die Erfinder haben sich die Aufgabe gestellt, neue Grundlagen für verbesserte Verfahren zur fermentativen Herstellung von L-Aminosäuren, insbesondere L-Lysin, mit coryneformen Bakterien bereitzustellen.

### Beschreibung der Erfindung

Werden im folgenden L-Aminosäuren oder Aminosäuren erwähnt, sind damit eine oder mehrere der proteinogenen Aminosäuren einschließlich ihrer Salze, ausgewählt aus der Gruppe L-Asparaginsäure, L-Asparagin, L-Threonin, L-Serin, L-Glutaminsäure, L-Glutamin, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Arginin und L-Prolin gemeint. Besonders bevorzugt ist L-Lysin.

Unter proteinogenen Aminosäuren versteht man die Aminosäuren die in natürlichen Proteinen, das heißt in Proteinen von Mikroorganismen, Pflanzen, Tieren und Menschen vorkommen.

Werden im Folgenden L-Lysin oder Lysin erwähnt, sind damit nicht nur die Basen, sondern sind auch die Salze wie z.B. Lysin-Monohydrochlorid oder Lysin-Sulfat gemeint.

Gegenstand der Erfindung ist ein Verfahren zur fermentativen Herstellung von L-Aminosäuren unter Verwendung von coryneformen Bakterien, die insbesondere bereits L-Aminosäuren produzieren und in denen mindestens eine oder mehrere der für die Gene amt, ocd, soxA und/oder sumT kodierende(n) Nukleotidsequenz(en) verstärkt, insbesondere überexprimiert oder auf hohem Niveau exprimiert wird (werden).

Gegenstand dieser Erfindung ist weiterhin ein Verfahren zur fermentativen Herstellung von L-Aminosäuren, in dem folgende Schritte durchführt werden:
a) Fermentation der L-Aminosäure produzierenden coryneformen bevorzugt rekombinanten Bakterien, in denen mindestens eines oder mehrere der Gene, ausgewählt aus der Gruppe amt, ocd, soxA und/oder sumT verstärkt, insbesondere überexprimiert oder auf hohem Niveau exprimiert wird (werden),
b) Anreicherung der L-Aminosäuren im Medium oder in den Zellen der Bakterien, und
c) Isolierung der gewünschten L-Aminosäuren, wobei gegebenenfalls Bestandteile der Fermentationsbrühe und/oder der Biomasse in Anteilen (> 0 bis 100 %) oder in ihren Gesamtmengen im Endprodukt verbleiben.

Die eingesetzten coryneformen Bakterien produzieren bevorzugt bereits vor der Verstärkung eines oder mehrerer der Gene amt, ocd, soxA und/oder sumT L-Aminosäuren, insbesondere L-Lysin.

Es wurde gefunden, dass coryneforme Bakterien nach Verstärkung eines oder mehrerer der Gene amt, ocd, soxA und/oder sumT in verbesserter Weise L-Aminosäuren, insbesondere L-Lysin, produzieren.

Die Genprodukte der drei in einem Operon angeordneten Gene amt, ocd und soxA sind mit dem Stickstoff-Metabolismus verknüpft.

Das Gen amt kodiert für den Ammonium-Transporter Amt in Corynebacterium glutamicum (Siewe et al., Journal of Biological Chemistry 271 (10): 5398-5402 (1996)), welcher abhängig von der internen Glutamin-, Glutaminanaloga- und NH₄⁺-Konzentration exprimiert wird und (Methyl-)Ammonium protonengetrieben in die Zelle transportiert (Meier-Wagner et al., Microbiology 147 (Pt 1): 135-143 (2001)).

Das Gen soxA kodiert für eine Sarcosin-Oxidase (Siewe et al., Journal of Biological Chemistry 271 (10): 5398-5402 (1996)). Sarkosinoxidasen gehören zu einer Gruppe von flavinhaltigen Oxidasen, die oxidative Reaktionen mit tertiären und sekundären Aminosäuren katalysieren und in Bacillus subtilis und Corynebacterium sp. P-1 ebenfalls Ammonium durch Deaminierung freisetzen (Job et al., Journal of Biological Chemistry 277 (9): 6985-6993 (2002); Chlumsky et. al. ; Biochemistry 32 (41): 11132-11142 (1993)).

Das Gen ocd kodiert für eine Ornithin-Cyclodeaminase (Jakoby et al.,FEMS Microbiology Letters 173 (2): 303-310 (1999)). Ornithin-Cyclodeaminasen katalysieren in Pseudomonaden den Abbau von Citrullin und Arginin zu Ornithin und setzten somit Ammonium in Form von Harnstoff bzw. Carbamoyl-Phosphat frei (Stalon et. al., Journal of General Microbiology 133 (PT9): 2487-2495 (1987)).

Das Gen sumT kodiert für eine Methyltransferase aus einer Gruppe von Uroporphyrin-III-C-Methyltransferasen (EC: 2.1.1.107), welche den Transfer von zwei Methylgruppen von S-Adenosyl-Methionin auf Uroporphyrinogen III katalysiert. Das Produkt Precorrin-2 ist ein Intermediat in der Biosynthese von Korrinoiden wie Cobalamin (Vitamin B12), Sirohäm, Häm_{d} oder Coenzym F430 (Raux et al., Cell Molecular Live Science 57 (13-14): 1880-1893 (2000)).

Die Nukleotidsequenzen der genannten Gene von Corynebacterium glutamicum gehören zum Stand der Technik und können verschiedenen Veröffentlichungen, Patentanmeldungen sowie der Datenbank des National Center for Biotechnology Information (NCBI) der National Library of Medicin (Bethesda, MD, USA) entnommen werden.
amt-Gen:

| | |
|---|---|
| Bezeichnung | Ammonium-Transporter Amt |
| Referenzen | Siewe et al., Journal. of Biological Chemistry 271 (10), 5398-5403 (1996); Sequenzen Nr. 3468 und Nr. 7064 aus EP1108790 |
| Accession No. | X93513, AX123552, AX127148 und AJ007732 |

ocd-Gen:

| | |
|---|---|
| Bezeichnung | putative Ornithin-Cyclodeaminase Ocd |
| Referenzen | Sequenzen Nr. 3467 und Nr. 7064 aus EP1108790 |
| Accession No. | AX123551, AX127148 und AJ007732 |

soxA-Gen:

| | |
|---|---|
| Bezeichnung | Sarcosin-Oxidase SoxA |
| Referenzen | Sequenzen Nr. 1748 und Nr. 7064 aus EP1108790 |
| Accession No. | AX121832, AX127148 und AJ007732 |

sumT:

| | |
|---|---|
| Bezeichnung | Methyltransferase SumT |
| Referenzen | Sequenz Nr. 994 und Nr. 7062 aus EP1108790 |
| Accession No. | AX121978 und AX127146 |

Die in den angegebenen Textstellen beschriebenen Sequenzen kodierend für die Gene amt, ocd, soxA und/oder sumT können erfindungsgemäß verwendet werden. Weiterhin können Allele der genannten Gene verwendet werden, die sich aus der Degeneriertheit des genetischen Kodes oder durch funktionsneutrale Sinnmutationen ("sense mutations") ergeben.

Der Begriff "Verstärkung" bzw. "Verstärken" beschreibt in diesem Zusammenhang die Erhöhung der intrazellulären Aktivität oder Konzentration eines oder mehrerer Enzyme bzw. Proteine in einem Mikroorganismus, die durch die entsprechende DNA kodiert werden, indem man beispielsweise die Kopienzahl des Gens bzw. der Gene erhöht, einen starken Promotor oder ein Gen bzw. Allel verwendet, das für ein entsprechendes Enzym bzw. Protein mit einer hohen Aktivität kodiert und gegebenenfalls diese Maßnahmen kombiniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise der Aktivität oder Konzentration des Proteins im Ausgangs-Mikroorganismus erhöht.

Die Erhöhung der Proteinkonzentration ist über 1- und 2-dimensionale Proteingelauftrennung und anschließende optische Identifizierung der Proteinkonzentration mit entsprechender Auswertesoftware im Gel nachweisbar. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2^{nd} Ed. Cold Spring Harbor Laboratory Press, Cold Spring Harbor, N.Y., 1989) und anschließende optische Auswertung mit entsprechender Software zur Konzentrationsbestimmung (Lohaus und Meyer (1998) Biospektrum 5:32-39; Lottspeich (1999) Angewandte Chemie 111:2630-2647) analysiert werden. Die Aktivität von DNA-bindenden Proteinen kann mittels DNA Band-Shift-Assays (auch als Gelretardation bezeichnet) gemessen werden (Wilson et al. (2001) Journal of Bacteriology 183:2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular cloning: a laboratory manual. 2^{nd} Ed. Cold Spring Harbor Laboratory Press, Cold Spring Harbor, N.Y., 1989).

Die Mikroorganismen, die Gegenstand der vorliegenden Erfindung sind, können Aminosäuren aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind besonders die bekannten Wildtypstämme
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Aminosäuren produzierende Mutanten bzw. Stämme wie beispielsweise die L-Lysin produzierenden Stämme
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM 5715
Stämme mit der Bezeichnung "ATCC" können von der American Type Culture Collection (Manassas, VA, USA) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden. Der genannte Stamm von Corynebacterium thermoaminogenes (FERM BP-1539) ist in der US-A-5.250.434 beschrieben.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen Aminosäure-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung. der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Zur Verstärkung wurden eines oder mehrere der Gene, ausgewählt aus der Gruppe amt, ocd, soxA und sumT beispielhaft mit Hilfe von episomalen Plasmiden überexprimiert. Als Plasmide eignen sich solche, die in coryneformen Bakterien repliziert werden. Zahlreiche bekannte Plasmidvektoren wie z.B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z.B. solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Reinscheid et al. (Applied and Environmental Microbiology 60, 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise pSUP301 (Simon et al., Bio/Technology 1, 784-791 (1983)), pK18mob oder pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-A 5,487,993), pCR®Blunt (Firma Invitrogen, Groningen, Niederlande; Bernard et al., Journal of Molecular Biology, 234: 534-541 (1993)), pEM1 (Schrumpf et al, 1991, Journal of Bacteriology 173:4510-4516) oder pBGS8 (Spratt et al.,1986, Gene 41: 337-342) in Frage. Der Plasmidvektor, der das zu amplifizierende Gen enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiology Letters 123, 343-347 (1994)) beschrieben. Nach homologer Rekombination mittels eines "cross over"-Ereignisses enthält der resultierende Stamm mindestens zwei Kopien des betreffenden Gens.

Eine gebräuchliche Methode, eine oder mehrere zusätzliche Kopien eines Gens von C. glutamicum in das Chromosom des gewünschten coryneformen Bakteriums einzubauen, ist die von Schwarzer und Pühler (Bio/Technology 9, 84-87 (1991)), Peters-Wendisch et al. (Microbiology 144, 915 - 927 (1998)) sowie in WO03/014330 und WO03/04037 beschriebene Methode der Gen-Verdopplung. Dazu wird die Nukleotidsequenz des gewünschten ORF's, Gens oder Allels, gegebenenfalls einschließlich der Expressions- und/oder Regulationssignale isoliert und zwei Kopien, bevorzugt in Tandem-Anordnung, in einen für C. glutamicum nicht replikativen Vektor wie beispielsweise pK18mobsacB oder pK19mobsacB (Jäger et al., Journal of Bacteriology 174: 5462-65 (1992))kloniert. Der Vektor wird anschließend durch Transformation oder Konjugation in das gewünschte coryneforme Bakterium überführt. Nach homologer Rekombination mittels eines ersten, Integration bewirkenden "cross-over"-Ereignisses und eines geeigneten zweiten, eine Excision bewirkenden "cross-over"-Ereignisses im Zielgen bzw. in der Zielsequenz erreicht man den Einbau der Mutation. Danach werden solche Bakterien isoliert, bei denen zwei Kopien des ORF's, Gens oder Allels am jeweiligen natürlichen Ort anstelle der ursprünglich vorhandenen singulären Kopie vorliegen. Dabei verbleibt am jeweiligen natürlichen Genort keine zur episomalen Replikation in Mikroorganismen befähigte/befähigende Nukleotidsequenz, keine zur Transposition befähigte/befähigende Nukleotidsequenz und keine Resistenz gegen Antibiotika vermittelnde Nukleotidsequenz.

Zusätzlich kann es für die Produktion von L-Aminosäuren vorteilhaft sein, zusätzlich zur Verstärkung eines oder mehrerer der Gene, ausgewählt aus der Gruppe amt, ocd, soxA und/oder sumT, eines oder mehrere Enzyme des jeweiligen Biosyntheseweges, der Glykolyse, der Anaplerotik, des Zitronensäure-Zyklus, des Pentosephosphat-Zyklus, des Aminosäure-Exports und gegebenenfalls regulatorische Proteine entweder zu verstärken, insbesondere überzuexprimieren, oder abzuschwächen, insbesondere die Expression zu verringern.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym bzw. Protein mit einer niedrigen Aktivität kodiert bzw. das entsprechende Gen oder Enzym (Protein) inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-. Proteins, beziehungsweise der Aktivität oder Konzentration des Proteins im Ausgangs-Mikroorganismus, herabgesenkt.

Die Verwendung endogener Gene wird im allgemeinen bevorzugt. Unter "endogenen Genen" oder "endogenen Nukleotidsequenzen" versteht man die in der Population einer Art vorhandenen Gene beziehungsweise Nukleotidsequenzen.

So kann beispielsweise für die Herstellung von L-Lysin neben der Verstärkung eines oder mehrerer der Gene, ausgewählt aus der Gruppe amt, ocd, soxA und/oder sumT, eines oder mehrere der Gene ausgewählt aus der Gruppe der Gene oder Allele der Lysinproduktion verstärkt, insbesondere überexprimiert werden. Unter "Gene oder Allele der Lysinproduktion sind sämtliche, bevorzugt endogene, offene Leserahmen, Gene oder Allele zu verstehen, deren Verstärkung/Überexpression eine Verbesserung der Lysinproduktion bewirken kann.

Hierzu gehören unter anderem folgende Gene oder Allele: accBC, accDA, cstA, cysD, cysE, cysH, cysK, cysN, cysQ, dapA, dapB, dapC, dapD, dapE, dapF, ddh, dps, eno, gap, gap2, gdh, gnd,lysC, lysC^{FBR}, lysE, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf und zwf A243T. Diese sind in Tabelle 1 zusammengefasst und erläutert.

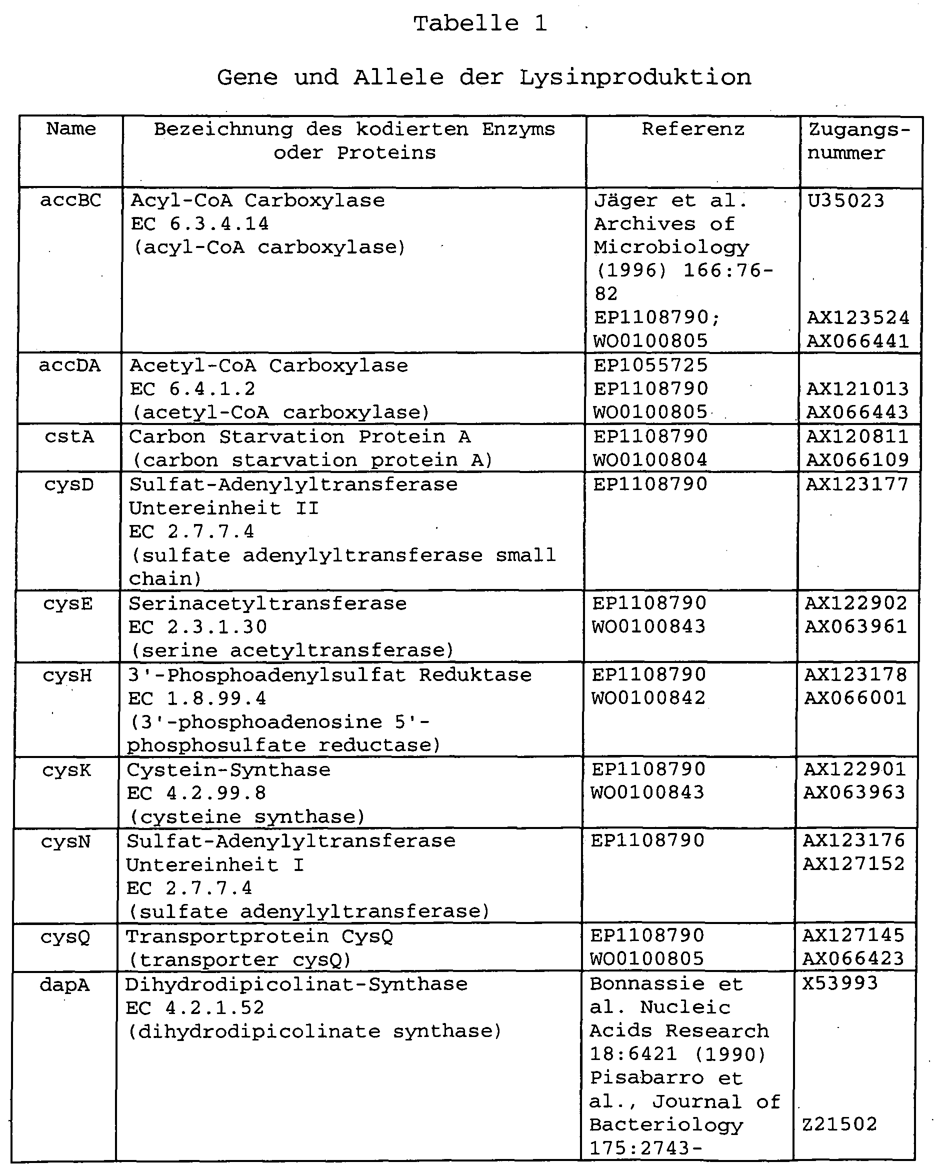

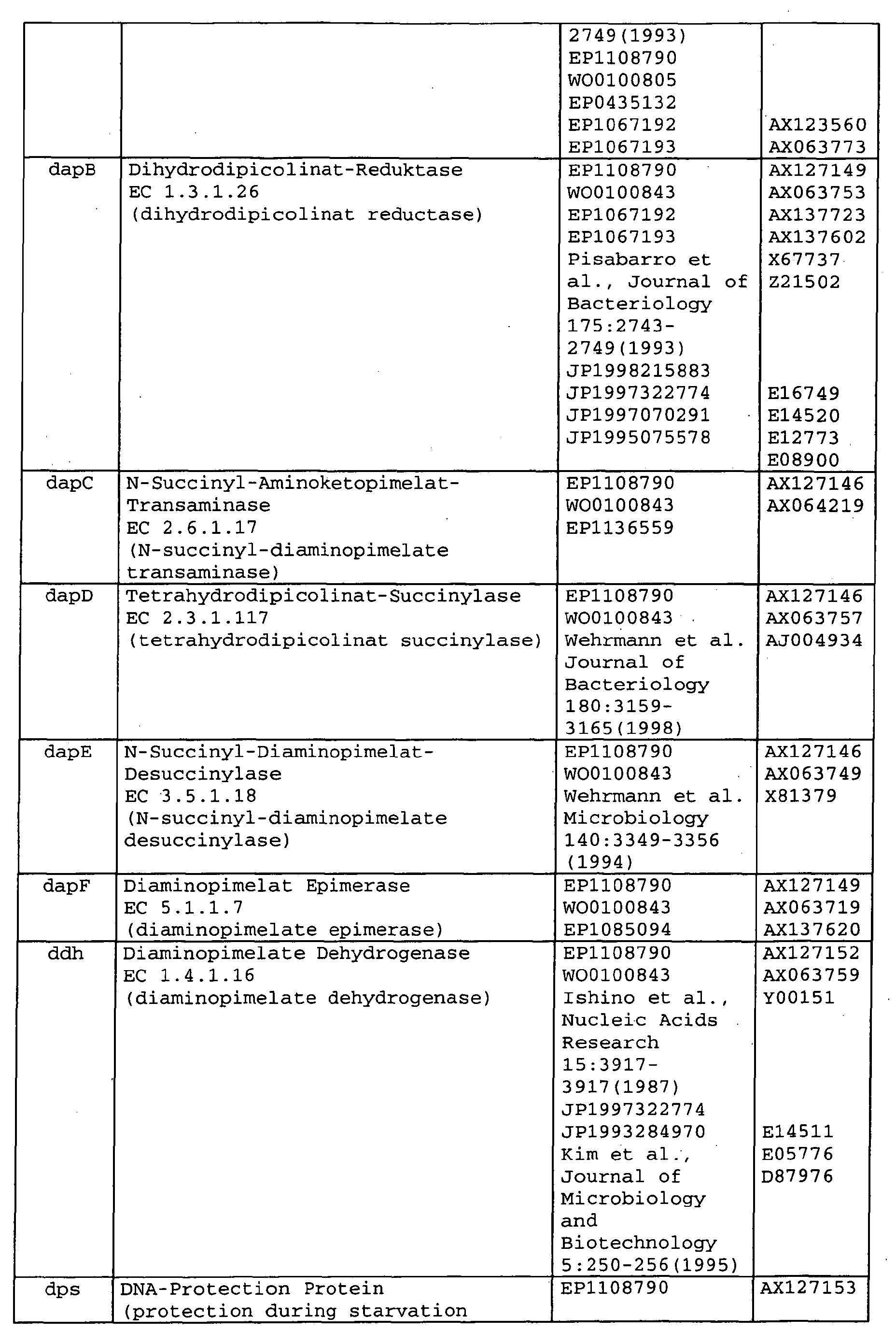

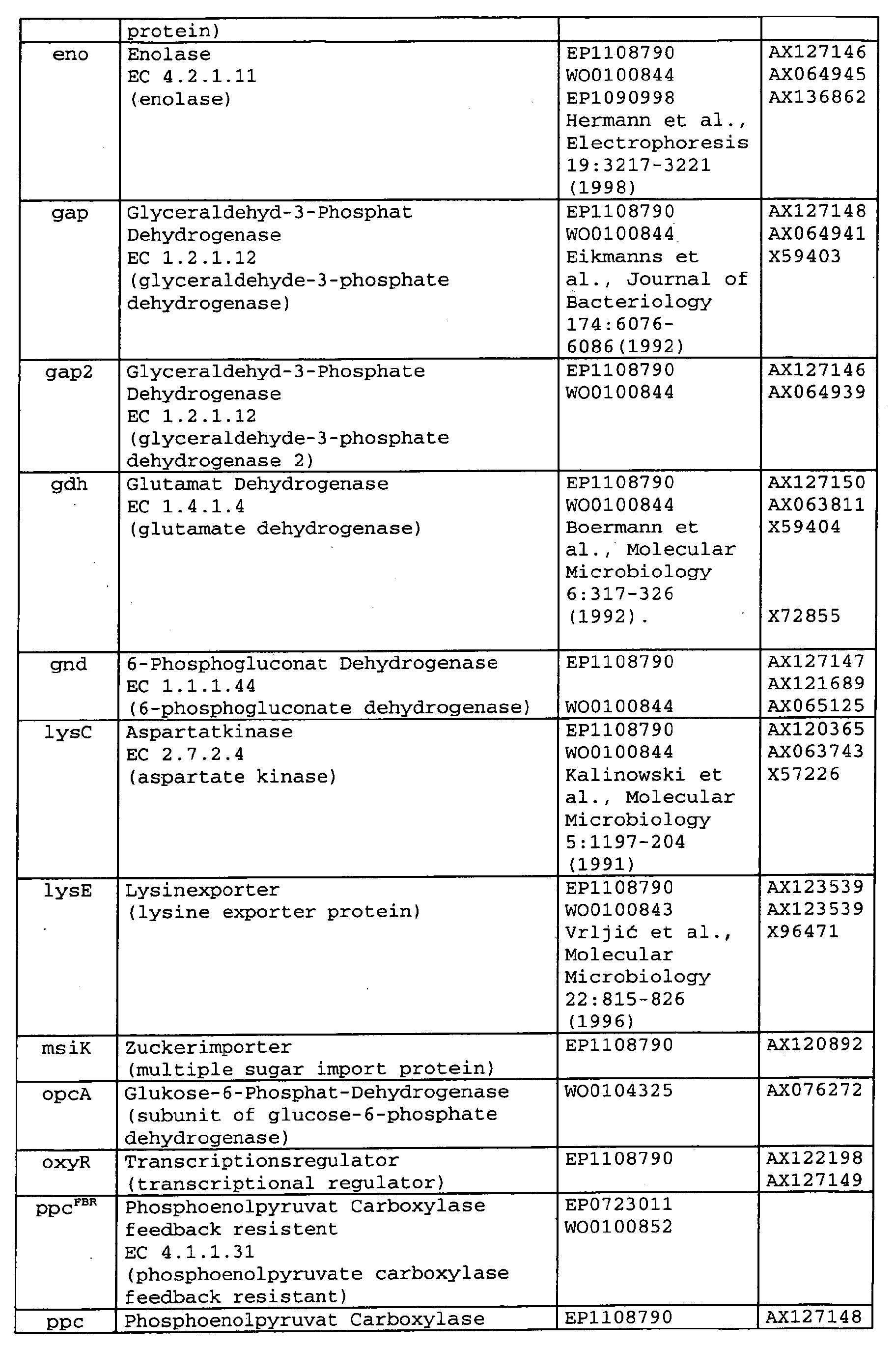

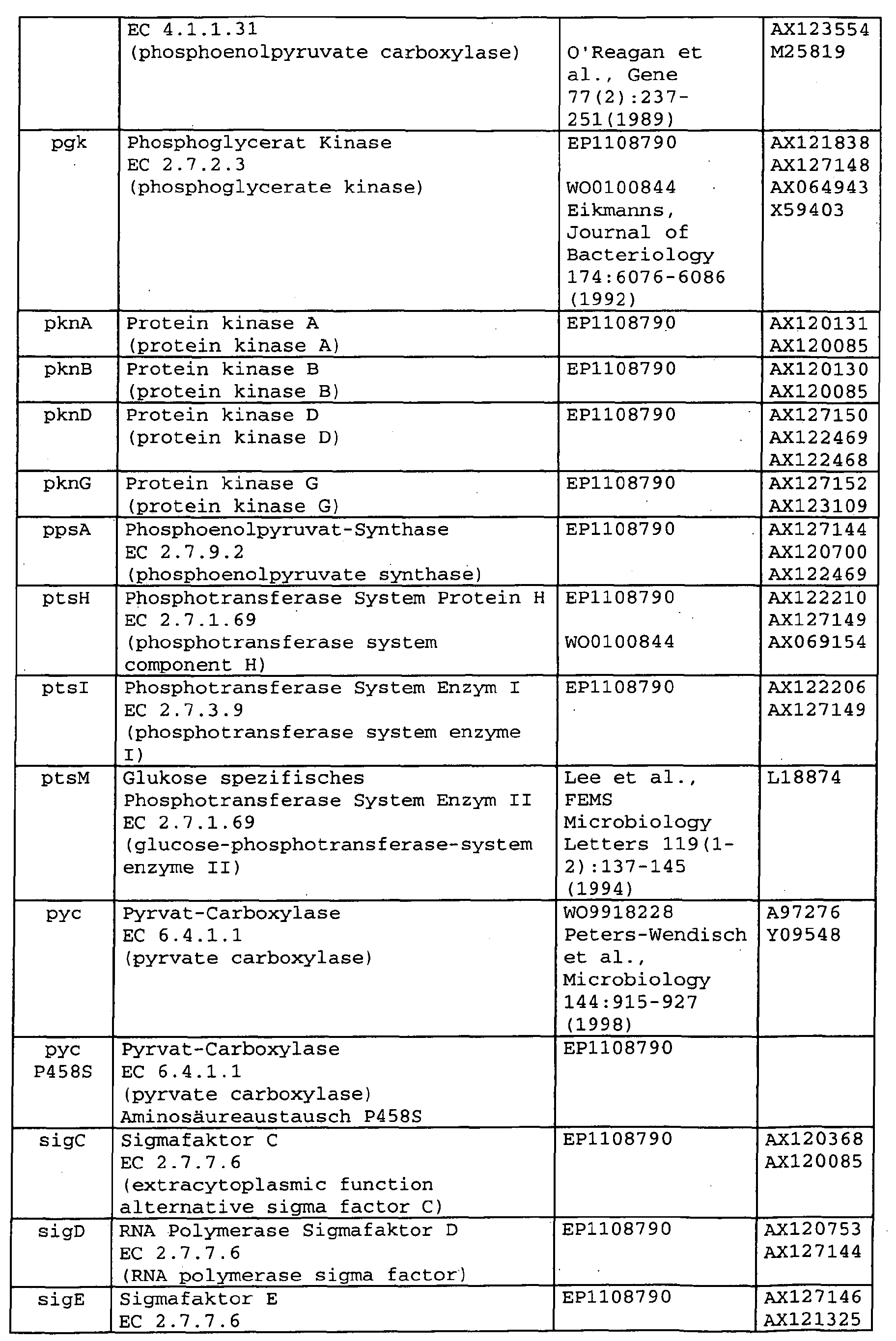

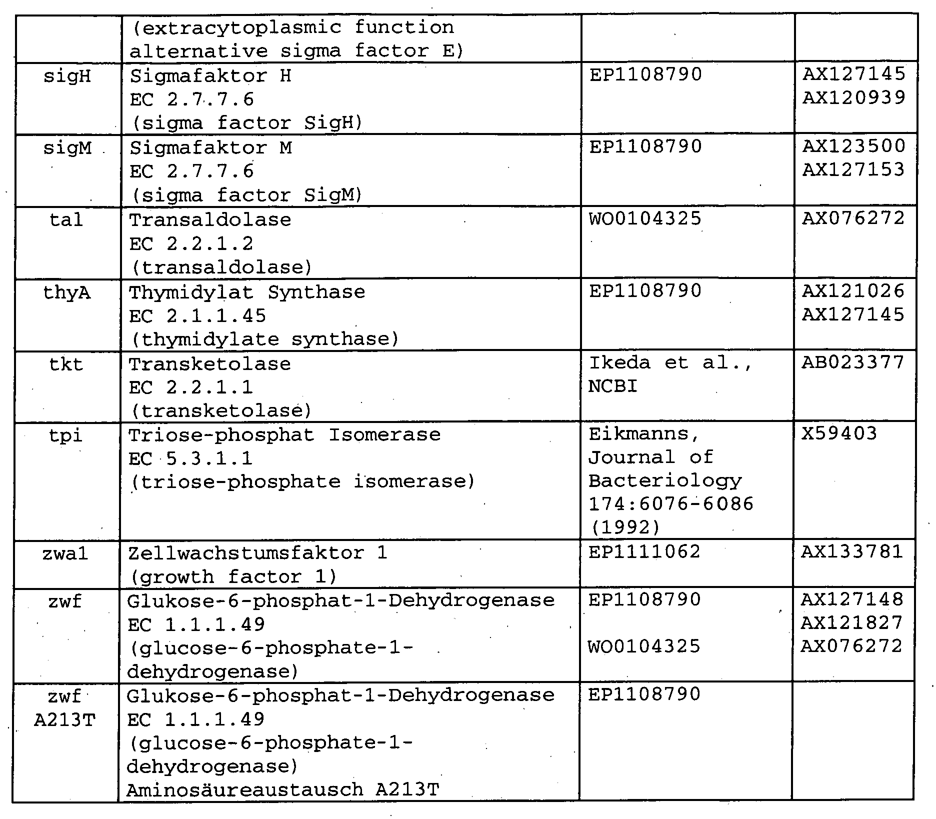

Weiterhin kann es für die Produktion L-Lysin vorteilhaft sein, zusätzlich zur Verstärkung eines oder mehrerer der Gene, ausgewählt aus der Gruppe amt, ocd, soxA und/oder sumT gleichzeitig eines oder mehrere der Gene, ausgewählt aus der Gruppe der Gene oder Allele, die für das Wachstum oder die Lysinproduktion nicht essentiell sind, abzuschwächen, insbesondere die Expression zu verringern.

Hierzu gehören unter anderem folgende offene Leserahmen, Gene oder Allele: aecD, ccpA1, ccpA2, citA, citB, citE, fda, gluA, gluB, gluC, gluD, luxR, luxS, lysR1, lysR2, lysR3, menE, mqo, pck, pgi, poxB und zwa2, welche in Tabelle 2 zusammengefasst und erläutert sind.

**Tabelle 2:**

| Gene und Allele, die für die Lysinproduktion nicht essentiell sind | | | |
|---|---|---|---|
| Genname | Bezeichnung des kodierten Enzyms oder Proteins | Referenz | Zugangs nummer |
| aecD | beta C-S Lyase | Rossol et al., Journal | M89931 |
| | EC 2.6.1.1 | of Bacteriology | |
| | (beta C-S lyase) | 174(9):2968-77 (1992) | |
| ccpA1 | Katabolit-Kontroll- | WO0100844 | AX065267 |
| | Protein | EP1108790 | AX127147 |
| | (catabolite control | WO 02/18419 | |
| | protein A1) | | |
| ccpA2 | Katabolit-Kontroll- | WO0100844 | AX065267 |
| | Protein | EP1108790 | AX121594 |
| | (catabolite control | | |
| | protein A2) | | |
| citA | Sensor-Kinase CitA | EP1108790 | AX120161 |
| | (sensor kinase CitA) | | |
| citB | Transkriptionsregulator | EP1108790 | AX120163 |
| | CitB | | |
| | (transcription regulator | | |
| | CitB) | | |
| citE | Citrat-Lyase | WO0100844 | AX065421 |
| | EC 4.1.3.6 | EP1108790 | AX127146 |
| | (citrate lyase) | | |
| fda | Fruktose-Bisphosphat- | von der Osten et al., | X17313 |
| | Aldolase | Molecular Microbiology | |
| | EC 4.1.2.13 | 3(11):1625-37 (1989) | |
| | (fructose 1,6- | | |
| | bisphosphate aldolase) | | |
| gluA | Glutamat-Transport ATP- | Kronemeyer et al., | X81191 |
| | bindendes Protein | Journal of Bacteriology | |
| | (glutamate transport | 177(5):1152-8 (1995) | |
| | ATP-binding protein) | | |
| gluB | Glutamat-bindendes | Kronemeyer et al., | X81191 |
| | Protein | Journal of Bacteriology | |
| | (glutamate binding | 177(5):1152-8 (1995) | |
| | protein) | | |
| gluC | Glutamat-Transport | Kronemeyer et al., | X81191 |
| | Permease | Journal of Bacteriology | |
| | (glutamate transport | 177(5):1152-8 (1995) | |
| | system permease) | | |
| gluD | Glutamat-Transport | Kronemeyer et al., | X81191 |
| | Permease | Journal of Bacteriology | |
| | (glutamate transport | 177(5):1152-8 (1995) | |
| | system permease) | | |
| luxR | Transkriptionsregulator | WO0100842 | AX065953 |
| | LuxR | EP1108790 | AX123320 |
| | (transcription regulator | | |
| | LuxR) | | |
| luxS | Histidin-Kinase LuxS | EP1108790 | AX123323 |
| | (histidine kinase LuxS) | | AX127153 |
| lysR1 | Transkriptionsregulator | EP1108790 | AX064673 |
| | LysR1 | | AX127144 |
| | (transcription regulator | | |
| | LysR1) | | |
| lysR2 | Transkriptionsaktivator | EP1108790 | AX123312 |
| | LysR2 | | |
| | (transcription regulator | | |
| | LysR2) | | |
| lysR3 | Transkriptionsregulator | WO0100842 | AX065957 |
| | LysR3 | EP1108790 | AX127150 |
| | (transcription regulator | | |
| | LysR3) | | |
| menE | O-Succinylbenzoesäure- | WO0100843 | AX064599 |
| | CoA-Ligase | EP1108790 | AX064193 |
| | EC 6.2.1.26 | | AX127144 |
| | (O-succinylbenzoate-CoA | | |
| | ligase) | | |
| mqo | Malat-Chinon- | Molenaar et al., Eur. | AJ224946 |
| | Oxidoreduktase | Journal of Biochemistry | |
| | (malate-quinone- | 1;254(2):395-403 (1998) | |
| | oxidoreductase) | | |
| pck | Phosphoenolpyruvat- | WO0100844 | AJ269506 |
| | Carboxykinase | EP-A-1094111 | AX065053 |
| | (phosphoenolpyruvate | | |
| | carboxykinase) | | |
| pgi | Glucose-6-Phosphat- | EP1087015 | AX136015 |
| | Isomerase | EP1108790 | AX127146 |
| | EC 5.3.1.9 | WO 01/07626 | |
| | (glucose-6-phosphate | | |
| | isomerase) | | |
| poxB | Pyruvat-Oxidase | WO0100844 | AX064959 |
| | EC 1.2.3.3 | EP1096013 | AX137665 |
| | (pyruvate oxidase) | | |
| zwa2 | Zellwachstumsfaktor 2 | EP1106693 | AX113822 |
| | (growth factor 2) | EP1108790 | AX127146 |

Schließlich kann es für die Produktion von Aminosäuren, vorteilhaft sein, neben der Verstärkung eines oder mehrerer der Gene, ausgewählt aus der Gruppe amt, ocd, soxA und/oder sumT unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen sind ebenfalls Gegenstand der Erfindung und können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Lactose, Fructose, Maltose, Melasse, Stärke und Cellulose, Öle und Fette wie z.B. Sojaöl, Sonnenblumenöl, Erdnußöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe wie z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Mit den Methoden der Erfindung kann die Leistung der Bakterien oder des Fermentationsprozesses bezüglich der Produkt-Konzentration ((Produkt pro Volumen), der ProduktAusbeute (gebildetes Produkt pro verbrauchter KohlenstoffQuelle), der Produkt-Bildung (gebildetes Produkt pro Volumen und Zeit) oder anderer Prozess-Parameter und Kombinationen davon um mindestens 0,5%, mindestens 1% oder mindestens 2% verbessert werden.

Methoden zur Bestimmung von L-Aminosäuren sind aus dem Stand der Technik bekannt. Die Analyse kann so wie bei Spackman et al. (Analytical Chemistry, 30, (1958), 1190) beschrieben durch Anionenaustauschchromatographie mit anschließender Ninhydrin Derivatisierung erfolgen, oder sie kann durch reversed phase HPLC erfolgen, so wie bei Lindroth et al. (Analytical Chemistry (1979) 51: 1167-1174) beschrieben.

### Folgende Figuren sind beigefügt:

Figur 1: Karte des Plasmids pVWEx1_amt_ocd_soxA
Figur 2: Karte des Plasmids pVWEx1_sumT

Bei der Angabe der Basenpaarzahlen handelt es sich um Näherungswerte, die im Rahmen der Reproduzierbarkeit von Messungen erhalten werden.

Die verwendeten Abkürzungen und Bezeichnungen haben folgende Bedeutung:

| | |
|---|---|
| amt_ocd_soxA | PCR-Fragment mit Ribosomen-Bindestelle und amt, ocd und soxA |
| sumT | PCR-Fragment mit Ribosomen-Bindestelle und sumT |
| Km | Kanamycin Resistenz-Gen |
| lacIq | lac Repressor-Gen lacI^{Q} |
| Ptac | tac-Promotor |
| SalI | Schnittstelle des Restriktionsenzyms SalI |
| XbaI | Schnittstelle des Restriktionsenzyms XbaI |
| BamHI | Schnittstelle des Restriktionsenzyms BamHI |

### Beispiel 1

Herstellung des Shuttlevektors pVWEx1-amt_ocd_soxA zur Verstärkung der Gene amt, ocd und soxA in C. glutamicum

### 1.1. Klonierung der Gene amt, ocd und soxA

Aus dem Stamm ATCC 13032 wird nach der Methode von Eikmanns et al. (Microbiology 140: 1817-1828 (1994)) chromosomale DNA isoliert. Aufgrund der für C. glutamicum bekannten Sequenz der Gene amt, ocd und soxA werden die folgenden Oligonukleotide für die Polymerase-Kettenreaktion ausgewählt. Zusätzlich werden eine geeignete Ribosomen-Bindestelle und geeignete Restriktionsschnittstellen eingefügt, die das Klonieren in den Zielvektor ermöglichen:

amt_ocd_soxA-frw (SEQ ID No. 1)
5` AC GC GTCGAC AAGGAGAAGGGC C ATG GAC CCC TCA GAT CTA G 3`

amt_ocd_soxA-rev (SEQ ID No. 2)
5` ACGC GTCGA CAC CGA GGG CAC ATC GGT G 3`

Die dargestellten Primer wurden von der MWG (Ebersbach, Deutschland) synthetisiert. Der Primer amt_ocd_soxA-frw enthält die Sequenz für die Schnittstelle der Restriktionsendonuklease Sall und der Primer amt_ocd_soxArev die Schnittstelle der Restriktionsendonuklease Sal1, die in der oben dargestellten Nukleotidabfolge durch Unterstreichen markiert sind. Die PCR-Reaktion wird nach der Standard-PCR-Methode von Innis et al. (PCR protocols. A guide to methods and applications, 1990, Academic Press) mit einem Gemisch aus Taq- und Tgo- Polymerase der Firma Roche Diagnostics GmbH (Mannheim, Deutschland) durchgeführt. Mit Hilfe der Polymerase-Kettenreaktion ermöglichen die Primer die Amplifikation eines 3393 bp großen DNA-Fragmentes, welches die Gene amt, ocd und soxA aus Corynebacterium glutamicum ohne potentielle Promotor-Region, aber mit einer eingefügten Ribosomen-Bindestelle trägt (SEQ ID No. 3). Das so amplifizierte Fragment wird in einem 1%igen Agarosegel elektrophoretisch geprüft.

Das auf diese Weise gewonnene PCR Fragment wird mit dem Restriktionsenzym Sal1 vollständig gespalten und nach Auftrennung in einem 1%tigem Agarosegel mit dem QiaExII Gel Extraction Kit (Product No. 20021, Qiagen, Hilden, Germany) aus dem Gel isoliert.

### 1.2. Klonierung der Gene amt, ocd und soxA in den Vektor pVWEx1

Als Basisvektor zur Expression sowohl in C. glutamicum als auch in E. coli wird der E. coli - C. glutamicum - Shuttle - Expressionsvektor pVWEx1 (Peters-Wendisch et al., Journal of Molecular Microbiology and Biotechnology 3(2): 295-300 (2001)) eingesetzt. DNA dieses Plasmids wird mit dem Restriktionsenzym Sal1 vollständig gespalten und anschließend mit shrimp alkalischer Phosphatase (Roche Diagnostics GmbH, Mannheim, Deutschland, Produktbeschreibung SAP, Product No. 1758250) dephosphoryliert. Das in Beispiel 1.1 aus dem Agarosegel isolierte Fragment, das die Gene amt, ocd, soxA sowie eine Ribosomen-Bindestelle trägt, wird mit dem so vorbereiteten Vektor pVWEx1 gemischt und der Ansatz mit T4-DNA-Ligase (Amersham Pharmacia, Freiburg, Deutschland) behandelt.

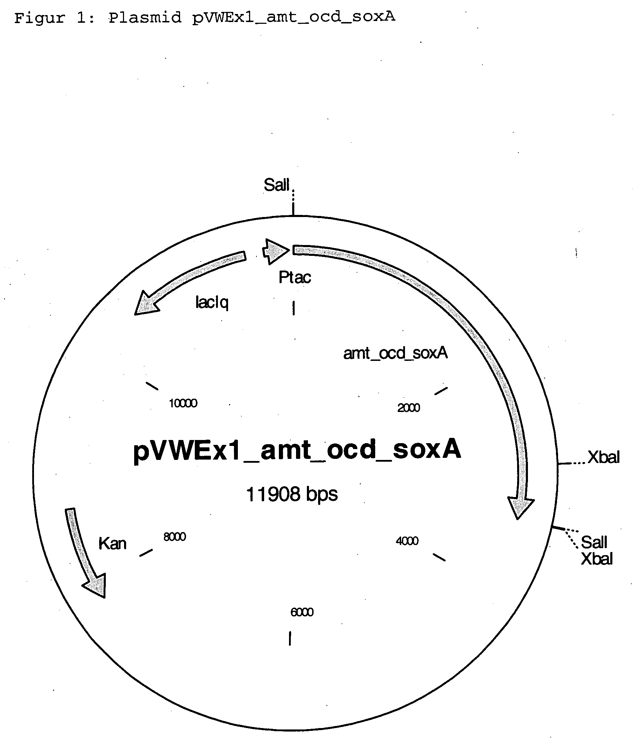

Der Ligationsansatz wird in den E. coli Stamm DH5αmcr (Hanahan, In: DNA Cloning. A Practical Approach. Vol. I. IRL-Press, Oxford, Washington DC, USA) transformiert. Die Selektion von Plasmid-tragenden Zellen erfolgte durch Ausplattieren des Transformationsansatzes auf LB-Agar (Lennox, 1955, Virology, 1:190) mit 50 mg/l Kanamycin. Nach Inkubation über Nacht bei 37°C werden rekombinante Einzelklone selektioniert. Plasmid DNA wird aus einer Transformante mit dem Qiaprep Spin Miniprep Kit (Product No. 27106, Qiagen, Hilden, Germany) nach Herstellerangaben isoliert und durch Restriktionsspaltung überprüft. Das erhaltene Plasmid wird pVWEx1-amt_ocd_soxA genannt. Es ist in Figur 1 dargestellt.

### Beispiel 2

### Transformation des Stammes DSM5715 mit dem Plasmid pVWEx1-amt_ocd_soxA

Um die Überlegenheit des beanspruchten Verfahrens zu demonstrieren, werden Versuche mit dem L-Lysin produzierenden Stamm Corynebacterium glutamicum DSM5715 (EP-B- 0435 132) durchgeführt. Dieser Stamm wurde durch Mutagenese von Corynebacterium glutamicum ATCC13032 mit Nitronitrosoguanidin entwickelt und enthält eine feedback resistente Aspartatkinase.

Der Stamm DSM5715 wird mit dem Plasmid pVWEx1-amt_ocd_soxA unter Anwendung der von Liebl et al., (FEMS Microbiology Letters, 53:299-303 (1989)) beschriebenen Elektroporationsmethode transformiert. Die Selektion der Transformanten erfolgt auf LBHIS Agar bestehend aus 18,5 g/l Brain-Heart Infusion Boullion, 0,5 M Sorbitol, 5 g/l Bacto-Trypton, 2,5 g/l Bacto-Yeast-Extract, 5 g/l NaCl und 18 g/l Bacto-Agar, der mit 25 mg/l Kanamycin supplementiert worden ist. Die Inkubation erfolgt für 2 Tage bei 30°C.

Plasmid DNA wird aus einer Transformante nach den üblichen Methoden isoliert (Peters-Wendisch et al., 1998, Microbiology, 144, 915 - 927), mit der Restriktionsendonuklease Sall geschnitten und das Plasmid durch anschließende Agarosegel-Elektrophorese überprüft. Der erhaltene Stamm wurde DSM5715/pVWEx1-amt_ocd_soxA genannt.

### Beispiel 3

### Herstellung von Lysin

Der in Beispiel 2 erhaltene C. glutamicum Stamm DSM5715/pVWEx1-amt_ocd_soxA wird in einem zur Produktion von Lysin geeigneten Nährmedium kultiviert und der Lysingehalt im Kulturüberstand bestimmt.

Dazu wird der Stamm zunächst auf Agarplatte mit dem entsprechenden Antibiotikum (LB-Agar mit Kanamycin (25 mg/l) für 24 Stunden bei 30°C inkubiert. Ausgehend von dieser Agarplattenkultur wird eine Vorkultur angeimpft (5 ml Medium im 10 ml Röhrchen). Als Medium für die Vorkultur wird das Vollmedium CgIII verwendet.

| Medium Cg III | |
|---|---|
| NaCl | 2,5 g/l |
| Bacto-Pepton | 10 g/l |
| Bacto-Yeast-Extrakt | 10 g/l |
| Glucose (getrennt autoklaviert) | 2% (w/v) |

Der pH-Wert wird auf pH 7.4
eingestellt

Diesem wird Kanamycin (25 mg/l) zugesetzt. Die Vorkultur wird 16 Stunden bei 30°C bei 180 rpm auf dem Schüttler inkubiert. Von dieser Vorkultur wird eine zweite Vorkultur angeimpft (50 ml Medium im 500 ml Erlenmeyerkolnben)und für 24h auf einem Schüttler bei 30°C bei 240rpm inkubiert. Als Medium für die zweite Vorkultur wird das Minimalmedium CgXII mit 10% (w/v) Glucose verwendet, welchem Kanamycin (25 mg/l) zugesetzt ist.

Von dieser zweiten Vorkultur wird eine Hauptkultur angeimpft, so daß die Anfangs-OD (600 nm) der Hauptkultur 0,5 OD beträgt. Für die Hauptkultur wird das Medium CGXII verwendet.

| Medium CGXII | |
|---|---|
| Harnstoff (Urea) | 5 g/l |
| MOPS (Morpholinopropansulfonsäure) | 42 g/l |
| Glucose (getrennt autoklaviert) | 100 g/l |
| Salze: | |
| (NH₄)₂SO₄) | 20 g/l |
| KH₂PO₄ | 1,0 g/l |
| K₂HPO₄ | 1,0 g/l |
| MgSO₄ * 7 H₂O | 0,25 g/l |
| CaCl₂ * 2 H₂O | 10 mg/l |
| FeSO₄ * 7 H₂O | 10 mg/l |
| MnSO₄ * H₂O | 10 mg/l |
| ZnSO₄ * 7 H2O | 1,0 mg/l |
| CuSO | 0,2 mg/l |
| NiCl₂ * 6H₂0 | 0,02 mg/l |
| Biotin (sterilfiltriert) | 0,2 mg/l |
| Protekatechuat (sterilfiltriert) | 0,03 mg/l |
| Leucin (sterilfiltriert) | 0,1 g/l |

Urea, MOPS und die Salzlösung werden mit KOH auf pH 7 eingestellt und autoklaviert, Die Glucoselösung wird separat autoklaviert. Anschließend werden die sterilen Substrat-, Aminosäure und Vitaminlösungen zugesetzt.

Die Kultivierung erfolgt in 50 ml Volumen in 500 ml Erlenmeyerkolben mit Schikanen. Es wird Kanamycin (25 mg/l) zugesetzt. Die Kultivierung erfolgt bei 30°C und 85% Luftfeuchtigkeit.

Nach 72 Stunden wird die OD bei einer Meßwellenlänge von 600 nm mit dem Biomek 1000 (Beckmann Instruments GmbH, München) ermittelt. Die gebildete Lysinmenge wird mittels HPLC (Flüssigchromatographie) mit einem Hewlet-Packard HPLC-Gerät TypHP1100 und o-Phtaldialdehyd-Derivatisierung mit einem Flureszenzdetektor G1321A bestimmt (Jones & Gilligan 1983).

In Tabelle 3 ist das Ergebnis des Versuchs dargestellt.

**Tabelle 3**

| Stamm | OD(600) | Lysin-HCl mM |
|---|---|---|
| DSM5715 | 30 | 76 |
| DSM5715/ pVWEx1-amt_ocd_soxA | 30 | 125 |

### Beispiel 4

Herstellung des Shuttlevektors pVWEx1-sumT zur Verstärkung des sumT-Gens in C. glutamicum

### 4.1. Klonierung des sumT-Gens

Aus dem Stamm ATCC 13032 wird nach der Methode von Eikmanns et al. (Microbiology 140: 1817-1828 (1994)) chromosomale DNA isoliert. Aufgrund der für C. glutamicum bekannten Sequenz des sumT-Gens werden die folgenden Oligonukleotide für die Polymerase-Kettenreaktion ausgewählt. Zusätzlich werden eine geeignete Ribosomen-Bindestelle und geeignete Restriktionsschnittstellen eingefügt, die das Klonieren in den Zielvektor ermöglichen:

sumT-frw (SEQ ID No. 4)
5` GC TCTAG AAGGAGATTCTCC ATG CAT GTT GCT GAA TTA TC 3`

sumTneu-rev (SEQ ID No. 5)
5` CG GGATC CGAT TAA TTT TCC CTG GCA G 3`

Die dargestellten Primer wurden von der MWG (Ebersberg, Deutschland) synthetisiert. Der Primer sumT-frw enthält die Sequenz für die Schnittstelle der Restriktionsendonuklease Xba1 und der Primer sumTneu-rev die Schnittstelle der Restriktionsendonuklease BamH1, die in der oben dargestellten Nukleotidabfolge durch Unterstreichen markiert sind. Die PCR-Reaktion wird nach der Standard-PCR-Methode von Innis et al. (PCR protocols. A guide to methods and applications, 1990, Academic Press) mit einem Gemisch aus Taq- und Tgo- Polymerase der Firma Roche Diagnostics GmbH (Mannheim, Deutschland) durchgeführt. Mit Hilfe der Polymerase-Kettenreaktion ermöglichen die Primer die Amplifikation eines 879 bp, welches das sumT-Gen aus Corynebacterium glutamicum ohne potentielle Promotor-Region trägt (SEQ ID No. 6). Das so amplifizierte Fragment wird in einem 1%igen Agarosegel elektrophoretisch geprüft.

Das auf diese Weise gewonnene PCR Fragment wird mit den Restriktionsenzymen Xba1 und BamH1 vollständig gespalten und nach Auftrennung in einem 1%igen Agarosegel mit dem QiaExII Gel Extraction Kit (Product No. 20021, Qiagen, Hilden, Germany) aus dem Gel isoliert.

### 4.2. Klonierung von sumT in den Vektor pVWEx1

Als Basisvektor zur Expression sowohl in C. glutamicum als auch in E. coli wird der E. coli - C. glutamicum - Shuttle - Expressionsvektor pVWEx1 (Peters-Wendisch et al., 2001) eingesetzt. DNA dieses Plasmids wird mit den Restriktionsenzymen Xba1 und BamH1 vollständig gespalten und anschließend mit shrimp alkalischer Phosphatase (Roche Diagnostics GmbH, Mannheim, Deutschland, Produktbeschreibung SAP, Product No. 1758250) dephosphoryliert. Das in Beispiel 4.1 aus dem Agarosegel isolierte sumT-Fragment wird mit dem so vorbereiteten Vektor pVWEx1 gemischt und der Ansatz mit T4-DNA-Ligase (Amersham Pharmacia, Freiburg, Deutschland) behandelt.

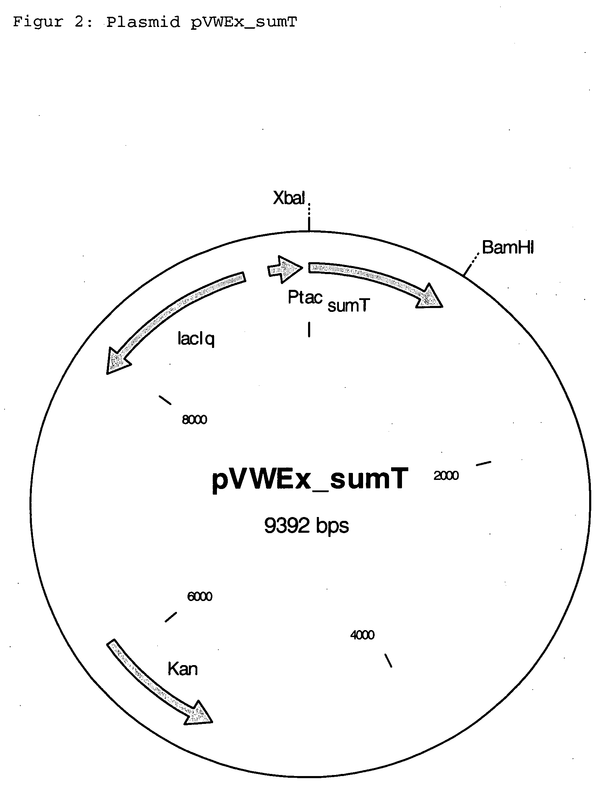

Der Ligationsansatz wird in den E. coli Stamm DH5αmcr (Hanahan, In: DNA Cloning. A Practical Approach. Vol. I. IRL-Press, Oxford, Washington DC, USA) transformiert. Die Selektion von Plasmid-tragenden Zellen erfolgte durch Ausplattieren des Transformationsansatzes auf LB-Agar (Lennox, 1955, Virology, 1:190) mit 50 mg/l Kanamycin. Nach Inkubation über Nacht bei 37°C werden rekombinante Einzelklone selektioniert. Plasmid DNA wird aus einer Transformante mit dem Qiaprep Spin Miniprep Kit (Product No. 27106, Qiagen, Hilden, Germany) nach Herstellerangaben isoliert und durch Restriktionsspaltung überprüft. Das erhaltene Plasmid wird pVWEx1-sumT genannt. Es ist in Figur 2 dargestellt.

### Beispiel 5

### Transformation des Stammes DSM5715 mit dem Plasmid pVWEx1-sumT

Der Stamm DSM5715 wird mit dem Plasmid pVWEx1-sumT unter Anwendung der von Liebl et al., (FEMS Microbiology Letters, 53:299-303 (1989)) beschriebenen Elektroporationsmethode transformiert. Die Selektion der Transformanten erfolgt auf LBHIS Agar bestehend aus 18,5 g/l Brain-Heart Infusion Boullion, 0,5 M Sorbitol, 5 g/l Bacto-Trypton, 2,5 g/l Bacto-Yeast-Extract, 5 g/l NaCl und 18 g/l Bacto-Agar, der mit 25 mg/l Kanamycin supplementiert worden ist. Die Inkubation erfolgt für 2 Tage bei 30°C.

Plasmid DNA wird aus einer Transformante nach den üblichen Methoden isoliert (Peters-Wendisch et al., 1998, Microbiology, 144, 915 - 927), mit den Restriktionsendonukleasen Xba1 und BamH1 geschnitten und das Plasmid durch anschließende Agarosegel-Elektrophorese überprüft.
Der erhaltene Stamm wurde DSM5715/pVWEx1-sumT genannt.

### Beispiel 6

### Herstellung von Lysin

Der in Beispiel 5 erhaltene C. glutamicum Stamm DSM5715/pVWEx1-sumT wird in einem zur Produktion von Lysin geeigneten Nährmedium kultiviert und der Lysingehalt im Kulturüberstand bestimmt.

Dazu wird der Stamm zunächst auf Agarplatte mit dem entsprechenden Antibiotikum (LB-Agar mit Kanamycin (25 mg/l) für 24 Stunden bei 30°C inkubiert. Ausgehend von dieser Agarplattenkultur wird eine Vorkultur angeimpft (5 ml Medium im 10 ml Röhrchen). Als Medium für die Vorkultur wird das Vollmedium CgIII verwendet.

| Medium Cg III | |
|---|---|
| NaCl | 2,5 g/l |
| Bacto-Pepton | 10 g/l |
| Bacto-Yeast-Extrakt | 10 g/l |
| Glucose (getrennt autoklaviert) | 2% (w/v) |

Der pH-Wert wird auf pH 7.4
eingestellt

Diesem wird Kanamycin (25 mg/l) zugesetzt. Die Vorkultur wird 16 Stunden bei 30°C bei 180 rpm auf dem Schüttler inkubiert. Von dieser Vorkultur wird eine zweite Vorkultur angeimpft (50 ml Medium im 500 ml Erlenmeyerkolnben)und für 24h auf einem Schüttler bei 30°C bei 240rpm inkubiert. Als Medium für die zweite Vorkultur wird das Minimalmedium CgXII mit 10% (w/v) Glucose verwendet, welchem Kanamycin (25 mg/l) zugesetzt ist.

Von dieser zweiten Vorkultur wird eine Hauptkultur angeimpft, so daß die Anfangs-OD (600 nm) der Hauptkultur 0,5 OD beträgt. Für die Hauptkultur wird das Medium CGXII verwendet.

| Medium CGXII | |
|---|---|
| Harnstoff (Urea) | 5 g/l |
| MOPS (Morpholinopropansulfonsäure) | 42 g/l |
| Glucose (getrennt autoklaviert) | 100 g/l |
| Salze: | |
| (NH₄)₂SO₄) | 20 g/l |
| KH₂PO₄ | 1,0 g/l |
| K₂HPO₄ | 1,0 g/l |
| MgS04 * 7 H₂O | 0,25 g/l |
| CaCl₂ * 2 H₂O | 10 mg/l |
| FeSO₄ * 7 H₂O | 10 mg/l |
| MnSO₄ * H₂O | 10 mg/l |
| ZnSO₄ * 7 H2O | 1,0 mg/l |
| CuSO | 0,2 mg/l |
| NiCl₂ * 6H₂0 | 0,02 mg/l |
| Biotin (sterilfiltriert) | 0,2 mg/l |
| Protekatechuat (sterilfiltriert) | 0,03 mg/l |
| Leucin (sterilfiltriert) | 0,1 g/l |

Urea, MOPS und die Salzlösung werden mit KOH auf pH 7 eingestellt und autoklaviert, Die Glucoselösung wird separat autoklaviert. Anschließend werden die sterilen Substrat-, Aminosäure und Vitaminlösungen zugesetzt.

Die Kultivierung erfolgt in 50 ml Volumen in 500 ml Erlenmeyerkolben mit Schikanen. Es wird Kanamycin (25 mg/l) zugesetzt. Die Kultivierung erfolgt bei 30°C und 85% Luftfeuchtigkeit.

Nach 72 Stunden wird die OD bei einer Meßwellenlänge von 600 nm mit dem Biomek 1000 (Beckmann Instruments GmbH, München) ermittelt. Die gebildete Lysinmenge wird mittels HPLC (Flüssigchromatographie) mit einem Hewlet-Packard HPLC-Gerät TypHP1100 und o-Phtaldialdehyd-Derivatisierung mit einem Flurescenzdetektor G1321A bestimmt (Jones & Gilligan 1983).

In Tabelle 4 ist das Ergebnis des Versuchs dargestellt.

**Tabelle 4**

| Stamm | OD(600) | Lysin-HCl mM |
|---|---|---|
| DSM5715 | 30 | 76 |
| DSM5715/pVWEx1-sumT | 30 | 128 |

## Patentansprüche

1. Verfahren zur Herstellung von L-Aminosäuren durch Fermentation coryneformer, insbesondere rekombinanter Bakterien, **dadurch gekennzeichnet, dass** man Bakterien einsetzt, in denen eines oder mehrere der mit dem Stickstoff-Metabolismus verknüpften Gene, ausgewählt aus der Gruppe amt, ocd, soxA und/oder sumT, verstärkt, insbesondere überexprimiert wird (werden).

2. Verfahren gemäß Anspruch 1, **dadurch gekennzeichnet, dass** die Konzentration oder Aktivität der durch die genannten Gene kodierten Proteine um jeweils 10 bis 2000 % gesteigert wird.

3. Verfahren gemäß Anspruch 1, **dadurch gekennzeichnet dass** man L-Lysin herstellt.

4. Verfahren zur fermentativen Herstellung von L-Aminosäuren, insbesondere L-Lysin,
**dadurch gekennzeichnet,**
**daß** man folgende Schritte durchführt:
a) Fermentation der die gewünschte L-Aminosäure produzierenden rekombinante coryneforme Bakterien, in denen man eines oder mehrere der Gene, ausgewählt aus der Gruppe amt, ocd, soxA und/oder sumT verstärkt, insbesondere überexprimiert;
b) Anreicherung des gewünschten Produkts im Medium oder in den Zellen der Bakterien, und
c) Isolierung der gewünschten L-Aminosäure, wobei gegebenenfalls Bestandteile der Fermentationsbrühe und/oder Biomasse in ihrer Gesamtheit oder in Anteilen (> 0 bis 100) im Endprodukt verbleiben.

5. Verfahren gemäß einem oder mehreren der Ansprüche 1 bis 4, **dadurch gekennzeichnet, daß** man Bakterien einsetzt, in denen man zusätzlich weitere Gene des Biosyntheseweges der gewünschten L-Aminosäure verstärkt, insbesondere überexprimiert.

6. Verfahren gemäß einem oder mehreren der Ansprüche 1 bis 5, **dadurch gekennzeichnet, daß** man Bakterien einsetzt, in denen die Stoffwechselwege zumindest teilweise ausgeschaltet sind, die die Bildung der gewünschten L-Aminosäure verringern.

7. Verfahren gemäß den Ansprüchen 1 oder 4, **dadurch gekennzeichnet, daß** man die Polynukleotide, die für eines oder mehrere der Gene, ausgewählt aus der Gruppe amt, ocd, soxA und/oder sumT kodieren, einzeln oder gemeinsam überexprimiert.

8. Verfahren gemäß den Ansprüchen 1 oder 4, **dadurch gekennzeichnet, daß** man die regulatorischen und/oder katalytischen Eigenschaften der Polypeptide (Enzymproteine) einzeln oder gemeinsam verstärkt, für die die Polynukleotide amt, ocd, soxA und/oder sumT kodieren.

9. Verfahren gemäß Anspruch 5, **dadurch gekennzeichnet, dass** die Aktivität oder Konzentration des (der) Protein(e), für das das (die) verstärkte(n) Gen(e) kodiert(en), um jeweils 10 bis 2000 % zunimmt.

10. Verfahren gemäß Anspruch 5 oder 9, **dadurch gekennzeichnet, daß** man für die Herstellung von L-Aminosäuren, insbesondere L-Lysin, coryneforme Mikroorganismen fermentiert, in denen man gleichzeitig eines oder mehrere der Gene, ausgewählt aus der Gruppe accBC, accDA, cstA, cysD, cysE, cysH, cysK, cysN, cysQ, dapA, dapB, dapC, dapD, dapE, dapF, ddh, dps, eno, gap, gap2, gdh, gnd,lysC, lysC^{FBR}, lysE, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf und zwf A243T verstärkt, insbesondere überexprimiert.

11. Verfahren gemäß Anspruch 6, **dadurch gekennzeichnet, dass** die Aktivität oder Konzentration des (der) Protein(e), für das das (die) abgeschwächte Gen(e) kodiert(en), auf jeweils 0 bis 75 % absinkt.

12. Verfahren gemäß Anspruch 6 oder 11, **dadurch gekennzeichnet, dass** man zur Herstellung von L-Aminosäuren, insbesondere L-Lysin, coryneforme Mikroorganismen fermentiert, in denen man gleichzeitig eines oder mehrere der Gene, ausgewählt aus der aecD, ccpA1, ccpA2, citA, citB, citE, fda, gluA, gluB, gluC, gluD, luxR, luxS, lysR1, lysR2, lysR3, menE, mqo, pck, pgi, poxB und zwa2 abschwächt, insbesondere ausschaltet oder die Expression verringert.

13. Verfahren gemäß einem oder mehreren der Ansprüche 1 - 12, **dadurch gekennzeichnet, dass** man Mikroorganismen der Art Corynebacterium glutamicum einsetzt.

14. Coryneforme Bakterien, in denen zumindest eines oder mehrere der Gene, ausgewählt aus der Gruppe amt, ocd, soxA und/oder sumT verstärkt, insbesondere überexprimiert ist (sind) vorliegt (en).


SEQUENCE LISTING

<110> Degussa AG

Forschungszentrum Jülich GmbH

<120> Verfahren zur fermentativen Herstellung von L-Aminosäuren unter
Verwendung coryneformer Bakterien

<130> 030263 BT

<160> 6

<170> PatentIn version 3.1

<210> 1
<211> 42
<212> DNA
<213> Künstliche Sequenz

<220>
<221> misc_feature
<222> (1)..(42)
<223> Primer amt_ocd_soxA-frw

<220>
<221> RBS
<222> (11)..(22)
<223> Ribosomen-Bindestelle

<400> 1
acgcgtcgac aaggagaagg gccatggacc cctcagatct ag 42

<210>  2

<211>  28

<212>  DNA

<213>  Künstliche Sequenz



<220>

<221>  misc_feature

<222>  (1)..(28)

<223>  Primer amt_ocd_soxA-rev



<400>  2
acgcgtcgac accgagggca catcggtg                                          28

<210>  3

<211>  3392

<212>  DNA

<213>  Künstliche Sequenz



<220>

<221>  misc_feature

<222>  (1)..(3393)

<223>  PCR-Produkt



<400>  3
acgcgtcgac aaggagaagg gccatggacc cctcagatct agcctggatt ctcgcagctt      60

ttgcgttggt aagcctgatg ttccccggat tgtccctgct ctacggcggc atgctgggtg     120

ggcaacacgt tcttaacacg ttcatgatgg ttatgagctc acttggaatc atcagccttg     180

tgtacatcat ttatggacac ggacttgtct taggaaactc catcggtggg tggggaatta     240

tcggaaatcc ccttgaatac ttcggcttcc gcaacattat ggaagatgac ggcaccggag     300

acctcatgtg ggccggcttc tacattctgt tcgctgcaat ctcactcgca cttgtttcat     360

ctggtgcagc ggggcgcatg cgctttggag cgtggctggt cttcggtgtc ctgtggttca     420

cctttgtgta cgcgccactg gcacactggg ttttcgctat cgatgatcct gagtccggct 480
acgtgggtgg ctggatgaaa aatgtgcttg agttccacga ctttgctggt ggaacggcag 540
tgcacatgaa tgcgggtgcg tctggactcg cgctggcaat agtgctggga cgccgccact 600
ccatggctgt gcgtccacac aaccttccac tgattttgat tggtgcagga ctgatcgttg 660
cgggctggtt cggattcaat ggtggtaccg caggtggtgc caacttcctc gcaagctacg 720
tggtcgttac ctctctcatt gctgcagctg gcggaatgat gggcttcatg ctcgttgaac 780
gtgtgttcag cggaaaaccc actttctttg gctcggcaac cggcacaatc gcaggccttg 840
tggctatcac cccggccgcg gatgcagtga gcccgctcgg agcattcgcc gtcggagcgc 900
tcggcgcagt tgtctccttc tgggcaatta gctggaagaa gggacaccga gtcgatgatt 960
ccttcgatgt gttcgcagtc cacggaatgg ccggcattgc aggtgcactg tttgtcatgc 1020
tctttggcga tccactagca ccagcgggag tttccggagt cttcttcggt ggcgaactct 1080
ccctgctgtg gagggaacca ctggccatca tcgtgaccct tacatacgca ttcggcgtga 1140
cctggttgat tgccacgatc ttgaacaagt tcatgactct gcgcatcacc tccgaagccg 1200
aatatgaagg cattgaccgc gcagaacacg cagaatctgc ctaccacctc aattccaacg 1260
gaattgggat ggcaacccgc accaatttcg gacctgaaat ccccgaggaa accgtgcccg 1320
acgccgtgca ggtgggcgtc gataagcaaa aaatcgctga tactcgaaag gcctcaaaat 1380
gaccgcaacc tacaccactg aaaccgccat caatttcttg ttcttgagcg aaccggacat 1440
gatcgcggcc ggagtcaaag acgtcgcgca atgcgtcgat gtcatggagg aaacgctcgt 1500
gctcttggcg cagggcgact acaaaatggc cggtttgaac tccaactcgc atggcgcgat 1560
gatcaccttc ccggaaaacc cagaatttga aggcatgccc aaggacggcc ccgaccgccg 1620
attcatggcg atgcccgcat acctcggcgg gcgattcaaa aacaccggcg tgaagtggta 1680
cggatccaac gcggaaaaca aggcctcagg cttgcctcgc tcgatccaca ccttcgtcct 1740
caacgacacg gtcaccggtg caccgaaggc catcatgtcc gcgaacctgc tgtccgccta 1800
ccgcaccggc gcggttcccg gcgtgggcgt gaagcactta gcggtcgccg acgcgacaac 1860
cttggctgtc gtcggacctg gtgtcatggc gaaaaccatc accgaagcgt gcatcgcaga 1920
gcgcccagga atcaccacca tcaagatcaa gggacgcagc gaacgcggca tcaacgcctt 1980
tgcaacatgg gcgttggaaa aattccccga gatcgaagtg gtcgccgtcg gatctgaaga 2040
agacgtggtc aaagacgccg acatcgtcat cgccgccacc accacggacg ccgccggctc 2100
ctccgccttc ccatacttca aaaaagaatg gctcaagccg ggcgcattgc tgctgcttcc 2160
agccgccggt cgcttcgacg acgcttattt gcttgacgac gcccgcctcg ttgttgacta 2220
catggggctc tacgaagcct gggcagaaga atacggccca caggcctacc aactactcgg 2280

cattccagga acccactggt acgacctggc gctgcaagga aaactcgacc ttgcaaagat 2340

ttcccagatt ggcgatatct gctccggcaa gctacccgga cgcaccaacg atgaggaaat 2400

catcctctat tccgtcggcg gcatgccagt agaagacgtc gcctgggcaa cccaagtgta 2460

tgaaaacgcc ctggaaaaag gcgtcggcac cacattgaac ctgtgggaat cacccgcact 2520

ggcttgagag aagaaacaac aatgaaaatt gcggtaatcg gccttggatc aaccggctcc 2580

atggcactgt ggcacttaag taacatccca ggtgtagagg ccatcggctt tgaacaattc 2640

ggcatctccc atggctacgg cgcattcaca ggggagtccc gactgtttcg catggcctac 2700

cacgaaggca gcacctacgt tccgttgctc aaacgcgcac gagcactatg gtcatcactg 2760

agcgagattt ccggacgcga actcttccac aacttcggtg tcttaagcac cggcaaggaa 2820

gacgaagcac ccttccaacg cctggtggaa tcagtggaac gttatgagct gccacatgaa 2880

cgacttaccg ccgcgcagat gcgcaagcgt tacccaggtc tagacttccg cgatgatgaa 2940

gctggaattg ttgatcttca aggtggagcc ctgcgccccg aactagcagt gttcagtgca 3000

atcgaaacag ccaaggcaaa tggtgcccaa gtacgcgatc accaaaaaat caccagcatc 3060

gaagacaacg gcgatcacgt agtcatccaa gcaggcgaag aaaccacaat cgtggaccgc 3120

gttatcgtca ccaccggcag ctggacaagc gagctcgtgc cctccatcgc gccactgctt 3180

gaagtgcgac gcctagtgct cacctggttc ctgcccaaca atccagtgga cttccaaccg 3240

gaaaacctgc catgcttcat ccgtgaccgt gatggcttcc acgtatttgg agcaccatgc 3300

gtcgatgggt acagcatcaa aattgccgga ttggatgagt ggggcgttcc attaagcctc 3360

gatccaccga tgtgccctcg gtgtcgacgc gt 3392

<210> 4

<211> 40

<212> DNA

<213> Künstliche Sequenz

<220>

<221> misc_feature

<222> (1)..(40)

<223> Primer sumT-frw

<220>

<221>  RBS

<222>  (8)..(20)

<223>  Ribosomen-Bindestelle

<400>  4
gctctagaag gagattctcc atgcatgttg ctgaattatc                                40

<210>  5

<211>  27

<212>  DNA

<213>  Künstliche Sequenz

<220>

<221>  misc_feature

<222>  (1)..(27)

<223>  Primer sumTneu-rev

<400>  5
cgggatccga ttaattttcc ctggcag                                              27

<210>  6

<211>  879

<212>  DNA

<213>  Künstliche Sequenz

<220>

<221>  misc_feature

<222>  (1)..(879)

<223>  PCR-Produkt

<400>  6
gctctagaag gagattctcc atgcatgttg ctgaattatc tttgcccact ggaattatta      60

tcgcggcgac tccgctcggc aacattgggg atgcgtctcc gcgcctggtc cacgcgcttg     120

ccaacgccac tgtggtagct gcggaggata cccgcaggac ggcgtccttg gctgctgcgt     180

tgggggtgga aattaagggg cagttggtct cgaactttga ccataatgaa caggcgcgcg 240
tcggcaagct tattgaagca gcgcgcacgg gcacggtgct ggtggtcagc gatgccggca 300
tgcctgtggt ttctgatccg ggttttgcgc ttatcgacgc cgcccacgac gcgaacattc 360
cggtcacctg cttccccggg ccgtcagctg tgccaactgc gttggcattg tcgggccttc 420
acgtgggccg ctttgccttc gacggtttcg cgccgcgcaa acaaggtgcg cgcaccacgt 480
ggttggagtc gttgaaaacc gaaaagcgcg cggtatgttt cttcgaatct cctcaccgca 540
tcgcagaaac cctggctcac gctgccgaag ttttaggtga acgacgcgta gcagtgtgcc 600
gtgaactgtc caaaacctac gaacaggtaa agcgtggaac cttgccagag ttggcagaat 660
gggcacaaga tggggtgcgt ggcgagatca ccgttgtcat cgaaggcgcg ggcgatatcg 720
cggccgacgt cgattcgctt atcgacgccg cccagcagcg cgtcgattcc ggcgagcggt 780
tgaaagcggt gtgcgcagac ctcgcgaaaa tccatggcgt gagcaaaaat gaactctacg 840
atgcggttat ttctgccagg gaaaattaat cggatcccg 879